# EUROPEAN PATENT SPECIFICATION

(11) **EP 1 708 717 B1**
(45) Date of publication and mention of the grant of the patent: **05.10.2011**
(21) Application number: 04779258.5
(22) Date of filing: 27.07.2004
(51) Int. Cl.: A61K 31/138, A61P 11/06, A61P 11/02

(54) **Atomoxetine for treatment of allergic rhinitis and asthma**
Atomoxetin zur Behandlung von allergischer Rhinitis und Asthma
L'atomoxétine pour le traitement de la rhinite allergique et l'asthme

(30) Priority: 28.07.2003 US 490644 P
(43) Date of publication of application: 11.10.2006
(73) Proprietor: Dunaway, Leslie Joe, Louisville, KY 40245 (US)
(72) Inventor: Dunaway, Leslie Joe, Louisville, KY 40245 (US)
(74) Representative: Engelhard, Maximilian
(86) International application number: PCT/US2004/024125
(87) International publication number: WO 2005/011583

(56) References cited:
- EP-A1- 0 919 236
- EP-A2- 1 267 859
- WO-A1-03/049724
- US-A1- 2004 014 510
- LANNY J ROSENWASSER: "Treatment of Allergic Rhinitis" AMERICAN JOURNAL OF MEDICINE, EXCERPTA MEDICA, INC, UNITED STATES, vol. 113, no. 9A, 16 December 2002 (2002-12-16), pages 17S-24S, XP007911368 ISSN: 0002-9343
- HENRY LIN ET AL: "Treatment of Allergic Asthma" AMERICAN JOURNAL OF MEDICINE, EXCERPTA MEDICA, INC, UNITED STATES, vol. 113, no. 9A, 16 December 2002 (2002-12-16), pages 8S-16S, XP007911369 ISSN: 0002-9343

## Description

### TECHNICAL FIELD

This invention relates to the treatment of allergic rhinitis and asthma.

### BACKGROUND

Atomoxetine, sometimes also called tomoxetine, is a selective norepinephrine reuptake inhibitor. One chemical designation for atomoxetine is (-)-N-Methyl-3-phenyl-3-(o-tolyloxy)-propylamine. For teachings relating to this composition, see, e.g., U.S. Patent Nos. 4,229,449 and 4,271,160. Syntheses of atomoxetine are described in U.S. Pat. Nos. 4,018,895, 4,194,009, 4,314,081, 4,777,291, and 6,541,668. Also in this connection, see U.S. Patent No. 6,008,412, regarding processes for resolving N-methyl-3(R,S)-hydroxy-3-phenylpropylamine.
In the art the symptoms of allergic rhinitis are treated with two major classes of agents: oral H₁ antihistamines and intranasal corticosteroids that may be used as mono-therapy or in combination. Intranasal cromolyn is used for preventing the allergic reaction in some patients. In patients with severe symptoms immunotherapy is recommended (Lanny J. Rosenwasser: "Treatment of Allergic Rhinits", American Journal of Medicine, Excerpta Medica, Inc, US, vol. 113, no. 9A, 2002-12-16, pages 17S-24S).
Allergic rhinitis is frequently associated with asthma. In the art asthma is treated with corticosteroids in combination with β-agonists. Alternative treatment uses corticosteroids in combination with either leucotriene receptor antagonist or theophylline. Cromolyn is recommended for mild asthma symptoms (Henry Lin, et al.: "Treatment of Allergic Asthma", American Journal of Medicine, Excerpta Medica, Inc, US, vol. 113, no. 9A, 2002-12-16, pages 8S-16S).

### SUMMARY OF INVENTION

However, it has now been found that atomoxetine is useful in the treatment of allergic rhinitis and/or asthma. The precise mechanism by which atomoxetine produces its therapeutic effects in allergic rhinitis and asthma is unknown, but, without wishing to be bound by theory, is thought to be related to inhibiting reuptake of norepinephrine in an area of the brain responsible for regulation of the reactivity of the airways of the upper respiratory and lower respiratory tracts through stabilization of the epithelial membrane which lines these tracts to curb the transport of allergens across this membrane. It is a well known fact that allergic rhinitis and asthma are associated with an increased reactivity of the airways on exposure to environmental allergens. Thus reduction of the airway reactivity with a therapeutic dose of atomoxetine will decrease or even totally eliminate the symptoms of allergic rhinitis and Asthma.

One embodiment of this invention is atomoxetine for use in treating allergic rhinitis in a mammal comprising administering a therapeutically effective amount of atomoxetine to a mammal in need of such treatment. Other embodiments of the invention include atomoxetine for use in treating asthma in a mammal comprising administering a therapeutically effective amount of atomoxetine to a mammal in need of such treatment, and atomoxetine for use in treating allergic rhinitis and asthma in a mammal comprising administering a therapeutically effective amount of atomoxetine to a mammal in need of such treatment.

### FURTHER DETAILED DESCRIPTION OF THE INVENTION

The compound relating to this invention is atomoxetine, which is a well-known drug, the chemical name of which is (R)-(-)-N-methyl-3-(2-methylphenoxy)-3-phenylpropylamine. It is regularly used as a salt, and pharmaceutically acceptable salts are included in the term atomoxetine as used here. As used herein, the term "pharmaceutically acceptable salt" refers to those salts which are, within the scope of sound medical judgment, suitable for use in contact with the tissues of humans and other mammals without undue toxicity, irritation, allergic response, and are commensurate with a reasonable benefit/risk ratio. Pharmaceutically acceptable salts are well known in the art. The HCl salt of atomoxetine is an especially preferred pharmaceutically acceptable salt of atomoxetine in the practice of this invention. The HCl salt of atomoxetine has the chemical formula:

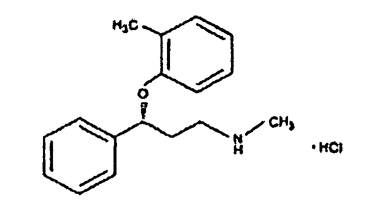

Allergic rhinitis is characterized by any combination of the symptoms of sneezing, clear nasal discharge, nasal congestion, clear postnasal drainage, and itchy, watery eyes occurring in a temporal relationship to allergen exposure. Following allergen exposure, the allergen is bound by Immunoglobulin E (IgE) molecules attached to a mast cell, which causes the release from the mast cell of many pharmacoactive compounds which lead to the production of allergy symptoms. Asthma is a disease of airways that is characterized by increased responsiveness of the tracheobronchial tree to a multiplicity of stimuli. Asthma is manifested physiologically by a widespread narrowing of the airways, and clinically by paroxysms of dyspnea, cough, and wheezing. As in allergic rhinitis, symptoms are initiated by binding of allergens to IgE attached to mast cells, causing the release from mast cells of many pharmacoactive compounds.

The effective dose of atomoxetine for the treatment of allergic rhinitis and/or asthma for humans is in the range from 5 mg/day to 100 mg/day. The preferred adult dose is in a range from 10 mg/day to 100 mg/day. A more preferred adult dose is in the range from 10 to 80 mg/day. The preferred children's dose is in a range from 5 mg/day to 60 mg/day, more preferable from 10 to 60 mg/day. Typically, the dose of atomoxetine is 1.2 to 1.4 mg of atomoxetine per kilogram of weight, but the optimum dose for each patient, as always, must be determined by the attending physician, taking into account other medications which the patient requires, severity of the illness, and all other pertinent, patient history, as well as the patient's weight.

Since atomoxetine is readily orally absorbed and requires only once/day administration, oral administration is highly preferred. Other methods of administration may be used when necessary, or when oral administration is inconvenient. Atomoxetine may be produced in the form of a clean, stable crystal, and thus is easily formulated in the usual oral pharmaceutical forms, such as tablets, capsules, and suspensions.

Atomoxetine of the present invention is effective in the treatment of both children and adults who suffer from symptoms of allergic rhinitis and/or asthma. Examples 1-4 demonstrate the effectiveness of atomoxetine in treating the symptoms of allergic rhinitis and asthma in humans.

### EXAMPLE 1

An 11 year old female with an 8 year history of allergic rhinitis and a 3 year history of moderate persistent asthma was given a 60 mg daily dose of atomoxetine. Within three weeks of initiation of treatment with atomoxetine, she was able to wean all traditional allergic rhinitis and asthma treatments (antihistamines, leukotriene modifiers, bronchodilators, and inhaled corticosteroids). During the following year, she was monitored while on atomoxetine mono therapy, and remained symptom free during the entire period of monitoring.

### EXAMPLE 2

A 51 year old with a 24 year history of severe allergic rhinitis, immunotherapy failure, and a 10 year history of moderate persistent asthma was given a 100 mg daily dose of atomoxetine. Within 10 days he was able to wean all traditional allergic rhinitis and asthma treatments (antihistamines, nasal corticosteroids, bronchodilators, and inhaled corticosteroids).
During the following 10 months, he was monitored while on atomoxetine mono therapy, and remained symptom free during the entire period of monitoring.

### EXAMPLE 3

A 25 year old female with a 20 year history of allergic rhinitis was given a 80 mg daily dose of atomoxetine. Within 7 days, she was able to wean all traditional allergic rhinitis (antihistamines and nasal corticosteroids). She has remained symptom free for at least nine months with atomoxetine mono therapy.

### EXAMPLE 4

A 40 year old female with a 10 year history of allergic rhinitis and a 5 year history of moderate persistent asthma was given a 80 mg daily dose of atomoxetine. Within 10 days, she was able to wean all traditional allergic rhinitis and asthma treatments (antihistamines, nasal corticosteroids, bronchodilators, theophylline, inhaled corticosteroids, and leukotriene modifiers). Her last pulmonary function test while on traditional treatments showed an FEV1 of 68% and repeat pulmonary function test after 6 weeks of atomoxetine mono therapy showed an FEV1 percent of 93%. She has remained symptom free for at least 7 months with atomoxetine mono therapy.

Here, as is known in the art, FEV1 percent is the ratio of FEV1 to the predicted FEV1, which is obtained from standardized tables based on age, weight, height, and sex; FEV1 is the volume of air that is forcefully exhaled from the lungs in one second.

## Claims

1. Atomoxetine for use in the treatment of allergic rhinitis in a mammal wherein a therapeutically effective amount of atomoxetine or pharmaceutically acceptable salt thereof is to be administered to a mammal in need of such treatment.

2. Atomoxetine for use in the treatment of allergic rhinitis according to Claim 1 wherein the pharmaceutically acceptable salt of atomoxetine is the HCI salt of atomoxetine.

3. Atomoxetine for use in the treatment of allergic rhinitis according to Claim 1 wherein the mammal is human.

4. Atomoxetine for use in the treatment of allergic rhinitis according to Claim 1 wherein the administering is by oral administration.

5. Atomoxetine for use in the treatment of allergic rhinitis according to Claim 1 wherein the pharmaceutically acceptable salt of atomoxetine is the HCI salt of atomoxetine, and wherein the mammal is human.

6. Atomoxetine for use in the treatment of allergic rhinitis according to Claim 1 wherein the pharmaceutically acceptable salt of atomoxetine is the HCI salt of atomoxetine, and wherein the administering is by oral administration.

7. Atomoxetine for use in the treatment of allergic rhinitis according to Claim 1 wherein the pharmaceutically acceptable salt of atomoxetine is the HCI salt of atomoxetine, wherein the mammal is human, and wherein the administering is by oral administration.

8. Atomoxetine for use in the treatment of asthma in a mammal wherein a therapeutically effective amount of atomoxetine or pharmaceutically acceptable salt thereof is to be administered to a mammal in need of such treatment.

9. Atomoxetine for use in the treatment of asthma according to Claim 8 wherein the pharmaceutically acceptable salt of atomoxetine is the HCI salt of atomoxetine.

10. Atomoxetine for use in the treatment of asthma according to Claim 8 wherein the mammal is human.

11. Atomoxetine for use in the treatment of asthma according to Claim 8 wherein the administering is by oral administration.

12. Atomoxetine for use in the treatment of asthma according to Claim 8 wherein the pharmaceutically acceptable salt of atomoxetine is the HCI salt of atomoxetine, and wherein the mammal is human.

13. Atomoxetine for use in the treatment of asthma according to Claim 8 wherein the pharmaceutically acceptable salt of atomoxetine is the HCI salt of atomoxetine, and wherein the administering is by oral administration.

14. Atomoxetine for use in the treatment of asthma according to Claim 8 wherein the pharmaceutically acceptable salt of atomoxetine is the HCI salt of atomoxetine, wherein the mammal is human, and wherein the administering is by oral administration.

15. Atomoxetine for use in the treatment of allergic rhinitis and asthma in a mammal wherein a therapeutically effective amount of atomoxetine or pharmaceutically acceptable salt thereof is to be administered to a mammal in need of such treatment.

16. Atomoxetine for use in the treatment of allergic rhinitis and asthma according to Claim 15 wherein the pharmaceutically acceptable salt of atomoxetine is the HCI salt of atomoxetine.

17. Atomoxetine for use in the treatment of allergic rhinitis and asthma according to Claim 15 wherein the mammal is human.

18. Atomoxetine for use in the treatment of allergic rhinitis and asthma according to Claim 15 wherein the administering is by oral administration.

19. Atomoxetine for use in the treatment of allergic rhinitis and asthma according to Claim 15 wherein the pharmaceutically acceptable salt of atomoxetine is the HCI salt of atomoxetine, and wherein the mammal is human.

20. Atomoxetine for use in the treatment of allergic rhinitis and asthma according to Claim 15 wherein the pharmaceutically acceptable salt of atomoxetine is the HCI salt of atomoxetine, and wherein the administering is by oral administration.

21. Atomoxetine for use in the treatment of allergic rhinitis and asthma according to Claim 15 wherein the pharmaceutically acceptable salt of atomoxetine is the HCI salt of atomoxetine, wherein the mammal is human, and wherein the administering is by oral administration.

## Patentansprüche

1. Atomoxetin zur Verwendung in der Behandlung von allergischer Rhinitis in einem Säuger, wobei eine therapeutisch wirksame Menge von Atomoxetin oder einem pharmazeutisch verträglichen Salz davon an einen Säuger, welcher einer solchen Behandlung bedarf, verabreicht wird.

2. Atomoxetin zur Verwendung in der Behandlung von allergischer Rhinitis gemäß Anspruch 1, wobei das pharmazeutisch verträgliche Salz von Atomoxetin das HCl-Salz von Atomoxetin ist.

3. Atomoxetin zur Verwendung in der Behandlung von allergischer Rhinitis gemäß Anspruch 1, wobei der Säuger ein Mensch ist.

4. Atomoxetin zur Verwendung in der Behandlung von allergischer Rhinitis gemäß Anspruch 1, wobei das Verabreichen durch orale Verabreichung stattfindet.

5. Atomoxetin zur Verwendung in der Behandlung von allergischer Rhinitis gemäß Anspruch 1, wobei das pharmazeutisch verträgliche Salz von Atomoxetin das HCl-Salz von Atomoxetin ist und wobei der Säuger ein Mensch ist.

6. Atomoxetin zur Verwendung in der Behandlung von allergischer Rhinitis gemäß Anspruch 1, wobei das pharmazeutisch verträgliche Salz von Atomoxetin das HCl-Salz von Atomoxetin ist und wobei das Verabreichen durch orale Verabreichung stattfindet.

7. Atomoxetin zur Verwendung in der Behandlung von allergischer Rhinitis gemäß Anspruch 1, wobei das pharmazeutisch verträgliche Salz von Atomoxetin das HCl-Salz von Atomoxetin ist, wobei der Säuger ein Mensch ist und wobei das Verabreichen durch orale Verabreichung stattfindet.

8. Atomoxetin zur Verwendung in der Behandlung von Asthma in einem Säuger, wobei eine therapeutisch wirksame Menge von Atomoxetin oder einem pharmazeutisch verträglichen Salz davon an einen Säuger, welcher einer solchen Behandlung bedarf, verabreicht wird.

9. Atomoxetin zur Verwendung in der Behandlung von Asthma gemäß Anspruch 8, wobei das pharmazeutisch verträgliche Salz von Atomoxetin das HCl-Salz von Atomoxetin ist.

10. Atomoxetin zur Verwendung in der Behandlung von Asthma gemäß Anspruch 8, wobei der Säuger ein Mensch ist.

11. Atomoxetin zur Verwendung in der Behandlung von Asthma gemäß Anspruch 8, wobei das Verabreichen durch orale Verabreichung stattfindet.

12. Atomoxetin zur Verwendung in der Behandlung von Asthma gemäß Anspruch 8, wobei das pharmazeutisch verträgliche Salz von Atomoxetin das HCl-Salz von Atomoxetin ist und wobei der Säuger ein Mensch ist.

13. Atomoxetin zur Verwendung in der Behandlung von Asthma gemäß Anspruch 8, wobei das pharmazeutisch verträgliche Salz von Atomoxetin das HCl-Salz von Atomoxetin ist und wobei das Verabreichen durch orale Verabreichung stattfindet.

14. Atomoxetin zur Verwendung in der Behandlung von Asthma gemäß Anspruch 8, wobei das pharmazeutisch verträgliche Salz von Atomoxetin das HCl-Salz von Atomoxetin ist, wobei der Säuger ein Mensch ist und wobei das Verabreichen durch orale Verabreichung stattfindet.

15. Atomoxetin zur Verwendung in der Behandlung von allergischer Rhinitis und Asthma in einem Säuger, wobei eine therapeutisch wirksame Menge von Atomoxetin oder einem pharmazeutisch verträglichen Salz davon an einen Säuger, welcher einer solchen Behandlung bedarf, verabreicht wird.

16. Atomoxetin zur Verwendung in der Behandlung von allergischer Rhinitis und Asthma gemäß Anspruch 15, wobei das pharmazeutisch verträgliche Salz von Atomoxetin das HCl-Salz von Atomoxetin ist.

17. Atomoxetin zur Verwendung in der Behandlung von allergischer Rhinitis und Asthma gemäß Anspruch 15, wobei der Säuger ein Mensch ist.

18. Atomoxetin zur Verwendung in der Behandlung von allergischer Rhinitis und Asthma gemäß Anspruch 15, wobei das Verabreichen durch orale Verabreichung stattfindet.

19. Atomoxetin zur Verwendung in der Behandlung von allergischer Rhinitis und Asthma gemäß Anspruch 15, wobei das pharmazeutisch verträgliche Salz von Atomoxetin das HCl-Salz von Atomoxetin ist und wobei der Säuger ein Mensch ist.

20. Atomoxetin zur Verwendung in der Behandlung von allergischer Rhinitis und Asthma gemäß Anspruch 15, wobei das pharmazeutisch verträgliche Salz von Atomoxetin das HCl-Salz von Atomoxetin ist und wobei das Verabreichen durch orale Verabreichung stattfindet.

21. Atomoxetin zur Verwendung in der Behandlung von allergischer Rhinitis und Asthma gemäß Anspruch 15, wobei das pharmazeutisch verträgliche Salz von Atomoxetin das HCl-Salz von Atomoxetin ist, wobei der Säuger ein Mensch ist und wobei das Verabreichen durch orale Verabreichung stattfindet.

## Revendications

1. Atomoxétine pour utilisation dans le traitement de la rhinite allergique chez un mammifère dans laquelle une quantité thérapeutiquement efficace d'atomoxétine ou d'un sel pharmaceutiquement acceptable de celle-ci est destinée à être administrée à un mammifère ayant besoin d'un tel traitement.

2. Atomoxétine pour utilisation dans le traitement de la rhinite allergique selon la revendication 1 dans laquelle le sel pharmaceutiquement acceptable d'atomoxétine est le sel HCl d'atomoxétine.

3. Atomoxétine pour utilisation dans le traitement de la rhinite allergique selon la revendication 1 dans laquelle le mammifère est un humain.

4. Atomoxétine pour utilisation dans le traitement de la rhinite allergique selon la revendication 1 dans laquelle l'administration est une administration par voie orale.

5. Atomoxétine pour utilisation dans le traitement de la rhinite allergique selon la revendication 1 dans laquelle le sel pharmaceutiquement acceptable d'atomoxétine est le sel HCl d'atomoxétine et dans laquelle le mammifère est un humain.

6. Atomoxétine pour utilisation dans le traitement de la rhinite allergique selon la revendication 1 dans laquelle le sel pharmaceutiquement acceptable d'atomoxétine est le sel HCl d'atomoxétine, et dans laquelle l'administration est une administration par voie orale.

7. Atomoxétine pour utilisation dans le traitement de la rhinite allergique selon la revendication 1 dans laquelle le sel pharmaceutiquement acceptable d'atomoxétine est le sel HCl d'atomoxétine, dans laquelle le mammifère est un humain, et dans laquelle l'administration est une administration par voie orale.

8. Atomoxétine pour utilisation dans le traitement de l'asthme chez un mammifère dans lequel une quantité thérapeutiquement efficace d'atomoxétine ou d'un sel pharmaceutiquement acceptable de celle-ci est destinée à être administrée à un mammifère ayant besoin d'un tel traitement.

9. Atomoxétine pour utilisation dans le traitement de l'asthme selon la revendication 8 dans laquelle le sel pharmaceutiquement acceptable de l'atomoxétine est le sel HCl d'atomoxétine.

10. Atomoxétine pour utilisation dans le traitement de l'asthme selon la revendication 8 dans laquelle le mammifère est un humain.

11. Atomoxétine pour utilisation dans le traitement de l'asthme selon la revendication 8 dans laquelle l'administration est une administration par voie orale.

12. Atomoxétine pour utilisation dans le traitement de l'asthme selon la revendication 8 dans laquelle le sel pharmaceutiquement acceptable d'atomoxétine est le sel HCl d'atomoxétine, et dans laquelle le mammifère est un humain.

13. Atomoxétine pour utilisation dans le traitement de l'asthme selon la revendication 8 dans laquelle le sel pharmaceutiquement acceptable d'atomoxétine est le sel HCl d'atomoxétine, et dans laquelle l'administration est une administration par voie orale.

14. Atomoxétine pour utilisation dans le traitement de l'asthme selon la revendication 8 dans laquelle le sel pharmaceutiquement acceptable d'atomoxétine est le sel HCl d'atomoxétine, dans laquelle le mammifère est un humain, et dans laquelle l'administration est une administration par voie orale.

15. Atomoxétine pour utilisation dans le traitement de la rhinite allergique et de l'asthme chez un mammifère dans laquelle une quantité thérapeutiquement efficace d'atomoxétine ou d'un sel pharmaceutiquement acceptable de celle-ci est destinée à être administrée à un mammifère ayant besoin d'un tel traitement.

16. Atomoxétine pour utilisation dans le traitement de la rhinite allergique et de l'asthme selon la revendication 15 dans laquelle le sel pharmaceutiquement acceptable d'atomoxétine est le sel HCl d'atomoxétine.

17. Atomoxétine pour utilisation dans le traitement de la rhinite allergique et de l'asthme selon la revendication 15 dans laquelle le mammifère est un humain.

18. Atomoxétine pour utilisation dans le traitement de la rhinite allergique et de l'asthme selon la revendication 15 dans laquelle l'administration est une administration par voie orale.

19. Atomoxétine pour utilisation dans le traitement de la rhinite allergique et de l'ashme selon la revendication 15 dans laquelle le sel pharmaceutiquement acceptable d'atomoxétine est le sel HCl d'atomoxétine, et dans laquelle le mammifère est un humain.

20. Atomoxétine pour utilisation dans le traitement de la rhinite allergique et de l'asthme selon la revendication 15 dans laquelle le sel pharmaceutiquement acceptable d'atomoxétine est le sel HCl d'atomoxétine, et dans laquelle l'administration est une administration par voie orale.

21. Atomoxétine pour utilisation dans le traitement de la rhinite allergique et de l'asthme selon la revendication 15 dans laquelle le sel pharmaceutiquement acceptable d'atomoxétine est le sel HCl d'atomoxétine, dans laquelle le mammifère est un humain, et dans laquelle l'administration est une administration pour voie orale.
